# EUROPÄISCHE PATENTSCHRIFT

(11) **EP 1 378 503 B1**
(45) Veröffentlichungstag und Bekanntmachung des Hinweises auf die Patenterteilung: **18.10.2006**
(21) Anmeldenummer: 03014665.8
(22) Anmeldetag: 27.06.2003
(51) Int. Cl.: C07C 69/68, C07C 67/62, C11D 17/00

(54) **Kompaktierter Milchsäurementhylester**
Compacted menthyl lactate
Lactate de menthyle compacte

(30) Priorität: 01.07.2002 DE 10229472
(43) Veröffentlichungstag der Anmeldung: 07.01.2004
(73) Patentinhaber: Symrise GmbH & Co. KG, 37603 Holzminden (DE)
(72) Erfinder: Körber, Alfred, 37603 Holzminden (DE)
(74) Vertreter: Eisenführ, Speiser & Partner

(56) Entgegenhaltungen:
- WO-A-95/07683
- WO-A-98/01134
- WO-A-98/02182
- WO-A-02/051392
- US-A- 3 064 311
- US-A- 5 783 725
- US-A- 5 843 466
- DATABASE CA [Online] CHEMICAL ABSTRACTS SERVICE, COLUMBUS, OHIO, US; KASHIWAGI, MITSUYOSHI ET AL: "Breath fresheners containing fatty acid esters or essential oils to mask saltiness of foaming agents" retrieved from STN Database accession no. 131:63259 XP002252645 & JP 11 152217 A (KAO CORP., JAPAN) 8. Juni 1999 (1999-06-08)
- DATABASE CA [Online] CHEMICAL ABSTRACTS SERVICE, COLUMBUS, OHIO, US; KUBO, KYOKO ET AL: "Oral resin compositions containing refreshing agents" retrieved from STN Database accession no. 138:276266 XP002252646 & JP 2003 095981 A (TAISHO PHARMACEUTICAL CO., LTD., JAPAN) 3. April 2003 (2003-04-03)
- DATABASE CA [Online] CHEMICAL ABSTRACTS SERVICE, COLUMBUS, OHIO, US; MISUMI, MANABU: "Granulated powder, manufacture of same and compression molded moldings of same." retrieved from STN Database accession no. 135:274893 XP002252647 & JP 2001 276597 A (KOBAYASHI PHARMACEUTICAL CO., LTD., JAPAN) 9. Oktober 2001 (2001-10-09)

## Beschreibung

Die Erfindung betrifft Milchsäurementhylester-Presslinge, ein Verfahren zur Herstellung von Milchsäurementhylester-Presslingen und deren Verwendung.

Milchsäurementhylester (MME, Menthyllactat) der Formel (1) ist eine Verbindung mit physiologischer Kühlwirkung auf Haut- und Schleimhäute des Körpers, die in einer Vielzahl von Produkten eingesetzt werden kann, bei denen eine lang anhaltende physiologische Kühlwirkung erwünscht wird wie z. B. in Genussmitteln wie Kaugummis, Kautabak, Zigaretten, Eis, Konfekt, Qetränken sowie in Körperpflegemitteln und pharmazeutischen oder kosmetischen Präparaten wie Zähnreinigungsmitteln, Mundwässern, Parfüms, Pudern, Lotionen, Salben, Ölen, Cremes, Lichtschutzzubereitungen, Rasiercremes und -wässer, Duschgels oder Shampoos. Vorteilhaft eingesetzt wird in diesen Produkten 1-Milchsäure-l-menthylester(1b).

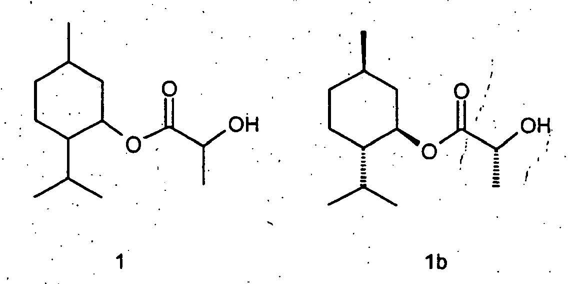

Der MME ist als erstarrtes Destillat oder auch als Kristallisat im Handel erhältlich. Beim Lagern über einen längeren Zeitraum, meist im Laufe mehrerer Wochen, entwickelt der MME einen säuerlichen, stechenden Geruch, wodurch dieser für die meisten Verwendungszwecke unbrauchbar wird. Zudem muss das feste Produkt vor der Dosierung und Einarbeitung in Produkte oder Artikel oft mehrfach aufgeschmolzen werden. Diese thermische Belastung mindert die Qualität des MME zusätzlich. Dieser Qualitätsverlust geht oftmals mit einem Ansteigen der Säurezahl einher.

MME wird üblicherweise durch säurekatalysierte Veresterung handelsüblicher Milchsäure mit handelsüblichem Menthol hergestellt, wie beispielsweise in EP-B 794 169 beschrieben. Zur weiteren Reinigung kann das Destillat einer Kristallisation unterworfen werden, das hierdurch erhältliche (1) weist meist eine Reinheit von größer 99,5 % (GC) auf.

Zur Vermeidung der säuerlichen, stechenden Note, kann beim Kristallisationsprozess ein Stabilisator in Form von Alkali- und/oder Erdalkalicarbonaten und/oder -hydrogencarbonaten zugesetzt werden, wie in EP-B 794169 beschrieben. Zur Erreichung der gewünschten Stabilisierung wird in diesem Falle in Gegenwart der genannten anorganischen Salze kristallisiert. Die durch dieses Verfahren erhältliche Mischung aus MME und anorganischem Salz ist bei manchen Anwendungen jedoch nachteilig, insbesondere wenn das anorganische Salz Inkompatibilitäten, Trübungen oder Niederschläge in der Anwendung bewirkt.

Die Aufgabe der vorliegenden Erfindung bestand nun darin eine Stabilisierung des Milchsäurementhylesters ohne den Zusatz anorganischer Salze zu erreichen.

Es wurde nun gefunden, dass durch. Pressung (Kompaktierung) des Milchsäurementhylesters gebildete Formkörper (Presslinge, Kompaktate) über einen langen Lagerzeitraum (mindestens 6 Monate) stabil sind und sich sensorisch nicht verändern. Die Säurezahl dieses gepressten Milchsäurementhylesters ändert sich während dieser Lagerzeit nicht.

Gegenstand der vorliegenden Erfindung sind daher Presslinge mit einem Gehalt an Milchsäurementhylester von mindestens 95 Gew.-%.

Ein weiterer Gegenstand der vorliegenden Erfindung ist ein Verfahren zur Herstellung von Milchsäurementhylester-Presslingen, gemäß Patentanspruch 5.

Bevorzugt ist ein Gehalt an MME im Pressling von mindestens 98 Gew.-%. In einer bevorzugten Ausführungsform liegt der Gehalt an MME im Pressling oberhalb von 99 Gew.-%. Die unterschiedlichen Reinheitsgrade können beispielsweise durch Destillation oder Kristallisation erhalten werden.

Erfindungsgemäß können alle Isomere des Milchsäurementhylesters in die Pressung eingesetzt werden, d.h. es können grundsätzlich die Milchsäureester von Menthol, Neomenthol, Isomenthol und/oder Neoisomenthol verwendet werden. Diese MME können als einzelne Isomere oder auch als beliebige Mischungen in die Pressung eingesetzt werden. Hierbei kann es sich um Menthylester der d-Milchsäure, der 1-Milchsäure oder einer beliebigen Mischung davon, wie z.B. der racemischen dl-Milchsäure, handeln. Vorteilhafte Milchsäureester sind solche des Menthols, bevorzugt des 1-Menthols. Ein ganz besonders bevorzugter MME ist 1-Michsäure-1-menthylester (1b).

Der MME wird bevorzugt in fester Form in die Pressung eingebracht, beispielsweise in Form von Kristallen, Schuppen, Granulaten, Pulvern oder Mischungen dieser Formen. Erfindungsgemäß bevorzugt sind Schuppen von MME. Die Schuppung des MME kann beispielsweise analog zu dem in US-A 3 064 311 beschriebenen Verfahren erfolgen, wobei ein flüssiger MME über eine gekühlte Schuppenwalze zu Schuppen geformt wird.

Erfindungsgemäß enthalten die MME-Presslinge keine Alkali- und/oder Erdalkalicarbonate und/oder -hydrogencarbonate als Stabilisatoren. Auch ein Zusatz anderer stabilisierend wirkender Stoffe ist nicht notwendig.

Die Presslinge können als solche in den Anwendungen eingesetzt werden, wodurch eine thermische Belastung durch Aufschmelzen vermieden wird. Des weiteren ist eine bequeme und sichere Handhabung der Presslinge bei der Einarbeitung in Formulierungen, Zubereitungen, Artikel und Produkte gegeben. Die Presslinge weisen eine gute Schütt- und Rieselfähigkeit auf, was für die Dosierung insbesondere im technischen und industriellen Bereich von Vorteil ist.

Die Form der Presslinge kann verschieden sein. Am gebräuchlichsten sind Kugeln, Würfel, Quader, Kissen, Zylinder, Tabletten, Pellets oder Briketts, eine bevorzugte Form ist das Pellet.

Die durch Kompaktierung gebildeten erfindungsgemäßen Presslinge bleiben bei der Lagerung über mehrere Monate formstabil. Ein Verklumpen, Verbacken oder Zusammenwachsen der Presslinge findet nicht statt. Eine gute Schütt- und Riesel-fähigkeit sowie Dosierbarkeit der erfindungsgemäßen Presslinge ist daher auch nach längeren Lagerzeiten gegeben.

Die Abmessungen des Presslings können stark differieren. Im Falle einer Kugel liegt der Durchmesser meist im Bereich 2 - 20 mm, bevorzugt bei 4 - 10 mm. Im Falle des Pellets liegen Länge und Breite meist bei 3 - 20 mm, bevorzugt bei 5 - 15 mm, die Höhe meist im Bereich von 2 - 15 mm, bevorzugt bei 5 - 12 mm.

Die Presskraft des Kompaktors liegt üblicherweise im Bereich von 10 - 100 kN (Kilo-Newton), bevorzugt bei 30 - 80 kN.

Bei der Kompaktierung des Milchsäurementhylesters sind auf den Durchmesser bezogen bevorzugt Linienpresskräfte von 1,5 - 4 Newton /mm·mm anzuwenden.

Bewährte Brikettierungsverfahren sind beispielsweise in Chemie - Anlagen + Verfahren 1985, Heft 4, Seiten 51,54 und 59 beschrieben.

Folgendes Beispiel erläutert die Erfindung.

### Beispiel

Über eine Schnecke mit anschließender Rüttelrinne werden einem Kompaktor der Firma BEPEX GmbH, bestehend aus zwei Kompaktorenwalzen (Ausmaße der Kompaktorwalzen: Breite 100 mm, Durchmesser 200 mm), pro Stunde 150 - 200 kg geschuppten Milchsäurementhylesters (GC-Gehalt: 97,3 % 1-Milchsäure-1-menthylester, 1,4 % d-Milchsäure-1-menthylester) zugeführt. Der Kompaktor erzeugte bei Raumtemperatur mit einer Presskraft von 50 kN Presslinge in Form von Pellets, Kissen oder Zylindern, mit den Ausmaßen Länge = Breite = 10 mm und Höhe = 6 mm.

## Patentansprüche

1. Milchsäurementhylester-Presslinge, **dadurch gekennzeichnet, dass** der Gehalt an Milchsäurementhylester im Pressling bei mindestens 95 Gew.-% liegt.

2. Presslinge nach Anspruch 1, **dadurch gekennzeichnet, dass** es sich um Presslinge aus 1-Milchsäure-1-menthylester handelt.

3. Presslinge nach mindestens einem der Ansprüche 1 bis 2, **dadurch gekennzeichnet, dass** geschuppter Milchsäurementhylester in die Pressung eingesetzt wird.

4. Presslinge nach mindestens einem der Ansprüche 1 bis 3, **dadurch gekennzeichnet, dass** diese in Form von Kugeln, Würfeln, Quadern, Kissen, Zylindem, Tabletten, Pellets oder Briketts vorliegen.

5. Verfahren zur Stabilisierung von Milchsäurementhylester mit einem Reinheitsgrad von mindestens 95 Gew.-%, mit folgendem Schritt:
Pressung des Milchsäurementhylesters zu einem Pressling.

6. Verfahren nach Anspruch 5, **dadurch gekennzeichnet, dass** in die Pressung geschuppter Milchsäurementhylester eingesetzt wird.

7. Verfahren nach mindestens einem der Ansprüche 5 bis 6, **dadurch gekennzeichnet, dass** 1-Milchsäure-1-menthylester eingesetzt wird.

8. Verfahren nach mindestens einem der Ansprüche 5 bis 7, **dadurch gekennzeichnet, dass** die Pressung mit einer Presskraft im Bereich von 10 - 100 kN durchgeführt wird.

9. Verwendung von Presslingen gemäß mindestens einem der Ansprüche 1 bis 4 zur Einarbeitung in Produkte.

## Claims

1. Menthyl lactate compacts, **characterised in that** the content of menthyl lactate in the compact is at least 95 wt.%.

2. Compacts according to claim 1, **characterised in that** it relates to compacts formed of 1-menthyl 1-lactate.

3. Compacts according to at least one of claims 1 to 2, **characterised in that** flaked menthyl lactate is placed in the press.

4. Compacts according to at least one of claims 1 to 3, **characterised in that** these are available in the shape of spheres, cubes, cuboids, cushions, cylinders, tablets, pellets or briquettes.

5. Process to stabilise menthyl lactate with a purity grade of at least 95 wt.%, by the following step:
press the menthyl lactate into a compact.

6. Process according to claim 5, **characterised in that** flaked menthyl lactate is placed in the press.

7. Process according to at least one of claims 5 to 6, **characterised in that** 1-menthyl 1-lactate is used.

8. Process according to at least one of claims 5 to 7, **characterised in that** compressing is carried out with a compression force in the range of 10-100 kN.

9. Use of compacts in accordance with at least one of claims 1 to 4 for working into products.

## Revendications

1. Comprimés de lactate de menthyle, **caractérisés en ce qu'**ils contiennent le lactate de menthyle à une concentration d'au moins 95 % en poids.

2. Comprimés selon la revendication 1, **caractérisés en ce qu'**il s'agit de comprimés de I-lactate de I-menthyle.

3. Comprimés selon au moins une des revendications 1 à 2, **caractérisés en ce que**, à la compression, on met en oeuvre du lactate de menthyle en écailles.

4. Comprimés selon au moins une des revendications 1 à 3, **caractérisés en ce qu'**ils présentent la forme de sphérules, de cubes, de primes, de coussins, de cylindres, de tablettes, de pellets ou de briquettes.

5. Procédé pour la stabilisation du lactate de menthyle à une pureté d'au moins 95 % en poids, comprenant le stade opératoire suivant :
formation d'un comprimé par compression du lactate de menthyle.

6. Procédé selon la revendication 5, **caractérisé en ce que**, pour la compression, on met en oeuvre du lactate de menthyle en écailles.

7. Procédé selon au moins une des revendications 5 à 6, **caractérisé en ce que** l'on met en oeuvre du I-lactate de I-menthyle.

8. Procédé selon au moins une des revendications 5 à 7, **caractérisé en ce que** la compression est réalisée sous une force de compression dans l'intervalle de 10 à 100 kN.

9. Utilisation de comprimés selon au moins une des revendications 1 à 4 pour l'incorporation dans des produits.
